# EUROPÄISCHE PATENTSCHRIFT

(11) **EP 3 779 495 B1**
(45) Veröffentlichungstag und Bekanntmachung des Hinweises auf die Patenterteilung: **11.10.2023**
(21) Anmeldenummer: 19192016.4
(22) Anmeldetag: 16.08.2019
(51) Int. Cl.: G01R 33/385, G01R 33/54, G01R 33/36

(54) **BILDGEBUNGSSYSTEM UMFASSEND EIN GRADIENTENSYSTEM MIT FLEXIBLER GRADIENTENVERSTÄRKEREINHEIT**
IMAGING SYSTEM COMPRISING A GRADIENT SYSTEM WITH A FLEXIBLE GRADIENT AMPLIFIER UNIT
SYSTÈME D'IMAGERIE AVEC UN SYSTÈME À GRADIENT POURVU D'UNITÉ FLEXIBLE D'AMPLIFICATEUR DE GRADIENT

(43) Veröffentlichungstag der Anmeldung: 17.02.2021
(73) Patentinhaber: Siemens Healthcare GmbH, 91052 Erlangen (DE)
(72) Erfinder: Hammes, Martin, 91054 Erlangen (DE)

(56) Entgegenhaltungen:
- EP-A1- 1 229 472
- DE-A1-102015 202 670
- DE-T2- 60 120 816
- US-A- 4 668 915
- R. KIMMLINGEN ET AL.: "An easy to exchange high performance head gradient insert for a 3T whole body MRI system: First results", PROCEEDINGS OF THE INTERNATIONAL SOCIETY FOR MAGNETIC RESONANCE IN MEDICINE, ISMRM, 12TH SCIENTIFIC MEETING AND EXHIBITION, KYOTO, JAPAN, 15-21 MAY 2004, 1. Mai 2004 (2004-05-01), XP040591571,

## Beschreibung

Die Erfindung betrifft ein Bildgebungssystem umfassend ein Gradientensystem und zwei Magnetresonanzgeräte. Des Weiteren betrifft die Erfindung ein Verfahren zu einer Ansteuerung eines erfindungsgemäßen Bildgebungssystems.

In einem Magnetresonanzgerät wird üblicherweise der zu untersuchende Körper eines Untersuchungsobjektes, insbesondere eines Patienten, mit Hilfe eines Hauptmagneten einem relativ hohen Hauptmagnetfeld, beispielsweise von 1,5 oder 3 oder 7 Tesla, ausgesetzt. Zusätzlich werden mit Hilfe einer Gradientenspuleneinheit Gradientenpulse ausgespielt. Über eine Hochfrequenzantenneneinheit werden dann mittels geeigneter Antenneneinrichtungen hochfrequente Hochfrequenz-Pulse, beispielsweise Anregungspulse, ausgesendet, was dazu führt, dass die Kernspins bestimmter, durch diese Hochfrequenz-Pulse resonant angeregter Atome um einen definierten Flipwinkel gegenüber den Magnetfeldlinien des Hauptmagnetfelds verkippt werden. Bei der Relaxation der Kernspins werden Hochfrequenz-Signale, so genannte Magnetresonanz-Signale, abgestrahlt, die mittels geeigneter Hochfrequenzantennen empfangen und dann weiterverarbeitet werden. Aus den so akquirierten Rohdaten können schließlich die gewünschten Bilddaten rekonstruiert werden.

Für eine bestimmte Messung ist daher eine bestimmte Magnetresonanz-Steuerungssequenz (MR-Steuerungssequenz), auch Pulssequenz genannt, auszusenden, welche aus einer Folge von Hochfrequenz-Pulsen, beispielsweise Anregungspulsen und Refokussierungspulsen, sowie passend dazu koordiniert auszusendenden Gradientenpulsen in verschiedenen Gradientenachsen entlang verschiedener Raumrichtungen besteht. Zeitlich passend hierzu werden Auslesefenster gesetzt, welche die Zeiträume vorgeben, in denen die induzierten Magnetresonanz-Signale erfasst werden.

Zum Erzeugen von Gradientenpulsen wird in die Gradientenspuleneinheit ein zeitlich variabler elektrischer Strom geleitet, dessen Amplitude bis zu 1,2 kA erreicht, wodurch ein Magnetfeldgradient, welcher für die räumliche Kodierung der Magnetresonanz-Signale essenziell ist, erzeugt wird. Der Magnetfeldgradient unterliegt Anstiegs- und Abfallraten von mehreren 100 T/m/s, was ein häufiges und rasches Wechseln der Stromrichtung erfordert. Die Ausgabe des zeitlich variablen elektrischen Stromes zur Ansteuerung einer Gradientenspuleneinheit erfolgt typischerweise zumindest zweistufig, wobei zunächst ein zeitlich variabler elektrischer Stellstrom ausgegeben wird, welcher Stellstrom durch eine Gradientenverstärkereinheit skaliert wird, sodass die erforderliche Amplitude erzielt wird. Je größer die Amplitude des Magnetfeldgradienten und/oder dessen Anstiegs- und Abfallraten sind, desto höher ist die erforderliche Leistung der Gradientenverstärkereinheit. Ein Magnetresonanzgerät wird abhängig vom zu erzeugenden Kontrast mit einer Vielzahl von verschiedenen MR-Steuerungssequenzen angesteuert. Typischerweise ist jedem Magnetresonanzgerät eine Gradientenverstärkereinheit direkt zugeordnet, welche zur Erzeugung aller vom Magnetresonanzgerät auszuspielenden MR-Steuerungssequenzen verwendet wird. Herkömmlich wird die einem Magnetresonanzgerät direkt zugeordnete Gradientenverstärkereinheit nur für dieses Magnetresonanzgerät verwendet.

DE 10 2015 202670 A1 beschreibt eine Magnetresonanzvorrichtung mit einer Schaltmatrixeinheit, die ausgebildet ist, zumindest einen Gradientenverstärker mit zumindest einer Gradientenspule flexibel zu verbinden.

DE 601 20 816 T2 betrifft ein Magnetresonanzbildgebungs-System, das einen elektronischen Leistungsschalter zwischen einem Gradientenverstärker und einer Gradientenspulenanordnung aufweist, welcher den Gradientenspulenstrom in kontinuierlicher Art und Weise leitet.

"An easy to exchange high performance head gradient insert for a 3T whole body MRI system: First results", Kimmlingen et al., ISMRM, 12TH SCIENTIFIC MEETING AND EXHIBITION, KYOTO, JAPAN, 2004, beschreibt eine Kopfgradientenspule für Diffusions-Tensor-Bildgebung.

EP 1 229 472 A1 offenbart ein Kommunikationsnetzwerk zwischen mehreren Magnetresonanzgeräten welche jeweils an unterschiedlichen Orten stehen.

US 4 668 915 A offenbart ein Magnetresonanzgerät mit zwei separaten Untersuchungsbereichen.

Der Erfindung liegt die Aufgabe zugrunde, ein besonders effizientes Bildgebungssystem für zumindest zwei Gradientenspuleneinheiten und damit für zumindest zwei Magnetresonanzgeräte anzugeben. Die Aufgabe wird durch die Merkmale der unabhängigen Ansprüche gelöst. Vorteilhafte Ausgestaltungen sind in den Unteransprüchen beschrieben.

Das erfindungsgemäße Bildgebungssystem umfasst ein Gradientensystem. Das Gradientensystem umfasst eine erste Gradientenspuleneinheit, eine zweite Gradientenspuleneinheit und eine flexible Gradientenverstärkereinheit, wobei die flexible Gradientenverstärkereinheit zu einer Ansteuerung der ersten Gradientenspuleneinheit und der zweiten Gradientenspuleneinheit ausgebildet ist.

Die erste Gradientenspuleneinheit ist dazu ausgebildet, einen ersten Magnetfeldgradienten in eine erste Richtung zu erzeugen. Die zweite Gradientenspuleneinheit ist dazu ausgebildet, einen zweite Magnetfeldgradienten in eine zweite Richtung zu erzeugen. Werden die erste Gradientenspuleneinheit und die zweite Gradientenspuleneinheit von zwei voneinander verschiedenen Magnetresonanzgeräten umfasst und/oder sind die erste Gradientenspuleneinheit und die zweite Gradientenspuleneinheit zwei voneinander verschiedenen Magnetresonanzgeräten zugeordnet, so kann die erste Richtung der zweiten Richtung entsprechen. Die erste Gradientenspuleneinheit kann einem ersten Magnetresonanzsystem zugeordnet sein. Die erste Gradientenspuleneinheit ist in diesem Fall typischerweise dazu ausgebildet, alle von dem ersten Magnetresonanzgerät auszugebenden Magnetfeldgradienten in die erste Richtung zu erzeugen. Die zweite Gradientenspuleneinheit kann einem zweiten Magnetresonanzsystem zugeordnet sein. Die zweite Gradientenspuleneinheit ist in diesem Fall typischerweise dazu ausgebildet, alle von dem zweiten Magnetresonanzgerät auszugebenden Magnetfeldgradienten in die zweite Richtung zu erzeugen.

Die flexible Gradientenverstärkereinheit ist typischerweise dazu ausgebildet, zu einem definierten Zeitpunkt die erste Gradientenspuleneinheit oder die zweite Gradientenspuleneinheit anzusteuern. Die flexible Gradientenverstärkereinheit ist typischerweise dazu ausgebildet, die erste Gradientenspuleneinheit und die zweite Gradientenspuleneinheit konsekutiv anzusteuern. Das Gradientensystem kann mehr als zwei Gradientenspuleneinheiten, insbesondere zumindest drei Gradientenspuleneinheiten, bevorzugt zumindest vier Gradientenspuleneinheiten umfassen. Die flexible Gradientenverstärkereinheit ist vorzugsweise mit allen von dem Gradientensystem umfassten Gradientenspuleneinheiten derart verbunden, dass die flexible Gradientenverstärkereinheit dazu ausgebildet ist, diese Gradientenspuleneinheiten anzusteuern.

Die flexible Gradientenverstärkereinheit umfasst typischerweise zumindest zwei Kondensatoren. Die flexible Gradientenverstärkereinheit umfasst typischerweise ein Kühlvorrichtung, welche zu einem Transport eines Kühlmediums ausgebildet ist. Die flexible Gradientenverstärkereinheit weist typischerweise zumindest einen Anschluss für einen Zufluss und/oder Abfluss eines Kühlmediums auf. Die flexible Gradientenverstärkereinheit weist typischerweise zumindest einen Anschluss an ein elektrisches Stromnetz auf.

Die flexible Gradientenverstärkereinheit weist typischerweise eine flexible Schnittstelle auf, welche mit einer ersten Schnittstelle von der ersten Gradientenspuleneinheit und mit einer zweiten Schnittstelle von der zweiten Gradientenspuleneinheit kompatibel ist. Die flexible Schnittstelle kann eine lösliche Verbindung mit der ersten Schnittstelle und/oder der zweiten Schnittstelle aufweisen.
Die flexible Gradientenverstärkereinheit kann zwei flexible Schnittstellen aufweisen, wovon jeweils eine mit der ersten Schnittstelle und eine mit der zweiten Schnittstelle verbindbar, vorzugsweise löslich verbindbar, ist. Die Anzahl der vom der flexiblen Gradientenverstärkereinheit umfassten flexiblen Schnittstellen entspricht typischerweise zumindest der Anzahl der mit der flexiblen Gradientenverstärkereinheit verbindbaren Gradientenspuleneinheiten.

Der Vorteil des Gradientensystems liegt darin, dass die flexible Gradientenverstärkereinheit nicht genau einer definierten Gradientenspuleneinheit und/oder genau einem definierten Magnetresonanzgerät zugeordnet ist. Die flexible Gradientenverstärkereinheit ist vielmehr zur Ansteuerung verschiedener Gradientenspuleneinheiten ausgebildet, welchen verschiedenen Gradientenspuleneinheiten verschiedene Magnetresonanzgeräte zugeordnet sind. Insbesondere kann die flexible Gradientenverstärkereinheit bei Bedarf mit weiteren Gradientenspuleneinheiten verbunden werden und/oder zur Ansteuerung weiterer Gradientenspuleneinheiten verwendet werden. Die flexible Gradientenverstärkereinheit kann demnach bedarfsgerecht verwendet werden. Dies ermöglicht eine effiziente Nutzung der flexiblen Gradientenverstärkereinheit und damit eine effiziente Nutzung des Gradientensystems.

Eine Ausführungsform des Gradientensystems sieht vor, dass das Gradientensystem eine Planungseinheit umfassend einen Planungseingang aufweist,
der Planungseingang dazu ausgebildet ist, eine erste Ansteuerungsinformation über eine Ansteuerung der ersten Gradientenspuleneinheit zu einem ersten Zeitpunkt und/oder eine zweite Ansteuerungsinformation über eine Ansteuerung der zweiten Gradientenspuleneinheit zu einem zweiten Zeitpunkt zu erfassen, und die Planungseinheit dazu ausgebildet ist, basierend auf der ersten Ansteuerungsinformation und/oder der zweiten Ansteuerungsinformation eine Zuweisung der flexiblen Gradientenverstärkereinheit zu dem ersten Zeitpunkt und/oder zu dem zweiten Zeitpunkt an die erste Gradientenspuleneinheit und/oder an die zweite Gradientenspuleneinheit vorzunehmen.

Der erste Zeitpunkt ist typischerweise ein zukünftiger Zeitpunkt. Der zweite Zeitpunkt ist typischerweise ein zukünftiger Zeitpunkt.

Eine Ansteuerung der ersten Gradientenspuleneinheit und/oder der zweiten Gradientenspuleneinheit umfasst ein Ausspielen von Gradientenpulsen im Rahmen einer MR-Steuerungssequenz. Eine erste MR-Steuerungssequenz umfasst typischerweise eine erste Ansteuerungsinformation über eine Ansteuerung der ersten Gradientenspuleneinheit zu einem ersten Zeitpunkt. Die erste MR-Steuerungssequenz sieht typischerweise die Ansteuerung eines der ersten Gradientenspuleneinheit zugeordneten ersten Magnetresonanzgerätes zu dem ersten Zeitpunkt vor. Die erste Ansteuerungsinformation kann typischerweise aus der ersten MR-Steuerungssequenz extrahiert werden.
Eine zweite MR-Steuerungssequenz umfasst typischerweise eine zweite Ansteuerungsinformation über eine Ansteuerung der zweiten Gradientenspuleneinheit zu einem zweiten Zeitpunkt. Die zweite MR-Steuerungssequenz sieht typischerweise die Ansteuerung eines der zweiten Gradientenspuleneinheit zugeordneten zweiten Magnetresonanzgerätes zu dem zweiten Zeitpunkt vor. Die zweite Ansteuerungsinformation kann typischerweise aus der zweiten MR-Steuerungssequenz extrahiert werden.

Die erste Ansteuerungsinformation und/oder die zweite Ansteuerungsinformation kann dem Planungseingang von einer Steuerungseinheit übermittelt werden, welche Steuerungseinheit Informationen hinsichtlich zukünftiger vom ersten und/oder zweiten Magnetresonanzgerät auszugebenden MR-Steuerungssequenzen umfasst. Das Erfassen der ersten Ansteuerungsinformation kann ein Bereitstellen der ersten MR-Steuerungssequenz und eine Extraktion der ersten Ansteuerungsinformation aus der ersten MR-Steuerungssequenz umfassen. Das Erfassen der zweiten Ansteuerungsinformation kann ein Bereitstellen der zweiten MR-Steuerungssequenz und eine Extraktion der zweiten Ansteuerungsinformation aus der zweiten MR-Steuerungssequenz umfassen.

Die Zuweisung der flexiblen Gradientenverstärkereinheit zu dem ersten Zeitpunkt an die erste Gradientenspuleneinheit umfasst typischerweise eine Initiierung einer Ansteuerung der ersten Gradientenspuleneinheit zum ersten Zeitpunkt durch die flexible Gradientenverstärkereinheit. Die Zuweisung der flexiblen Gradientenverstärkereinheit zu dem zweiten Zeitpunkt an die zweite Gradientenspuleneinheit umfasst typischerweise eine Initiierung einer Ansteuerung der zweiten Gradientenspuleneinheit zum zweiten Zeitpunkt durch die flexible Gradientenverstärkereinheit.

Die Planungseinheit ist typischerweise dazu ausgebildet, eine Verwendung der flexiblen Gradientenverstärkereinheit zu dem ersten Zeitpunkt und/oder zu dem zweiten Zeitpunkt festzulegen. Dabei wird typischerweise die erste Ansteuerungsinformation und/oder die zweite Ansteuerungsinformation dynamisch berücksichtigt.

Hierbei kann der Planungseinheit über den Planungseingang beispielsweise eine erste Ansteuerungsinformation für einen ersten Zeitraum umfassend den ersten Zeitpunkt für die erste Gradientenspuleneinheit bereitgestellt werden. Die erste Ansteuerungsinformation umfasst typischerweise die für die innerhalb des ersten Zeitraumes auszuspielenden Gradientenpulse im Rahmen einer ersten MR-Steuerungssequenz von einem der ersten Gradientenspuleneinheit zugeordneten ersten Magnetresonanzgerät.
Ist für die flexible Gradientenverstärkereinheit während des ersten Zeitraumes keine weitere Nutzung, also keine Ansteuerung einer anderen Gradientenspuleneinheit, beispielsweise der zweiten Gradientenspuleneinheit durch die flexible Gradientenverstärkereinheit vorgesehen, so wird die Planungseinheit typischerweise die flexible Gradientenverstärkereinheit der ersten Gradientenspuleneinheit zuweisen.

Der Planungseinheit können über den Planungseingang beispielsweise eine zweite Ansteuerungsinformation für einen zweiten Zeitraum umfassend den zweiten Zeitpunkt für die zweite Gradientenspuleneinheit bereitgestellt werden. Die zweite Ansteuerungsinformation umfasst typischerweise die für die innerhalb des zweiten Zeitraumes auszuspielenden Gradientenpulse im Rahmen einer zweiten MR-Steuerungssequenz von einem der zweiten Gradientenspuleneinheit zugeordneten zweiten Magnetresonanzgerät. Überlappt sich der erste Zeitraum mit dem zweiten Zeitraum, so kann die Planungseinheit dazu ausgebildet sein, unter Berücksichtigung weiterer Faktoren die flexible Gradientenverstärkereinheit der ersten Gradientenspuleneinheit für den ersten Zeitraum oder der zweiten Gradientenspuleneinheit für den zweiten Zeitraum zuzuweisen.

Die Planungseinheit kann beispielsweise dazu ausgebildet sein, die flexible Gradientenverstärkereinheit für das Ausspielen einer ersten MR-Steuerungssequenz zum ersten Zeitpunkt der ersten Gradientenspuleneinheit zuzuweisen. Dann wird die zweite Gradientenspuleneinheit zum ersten Zeitpunkt typischerweise nicht von der flexiblen Gradientenverstärkereinheit angesteuert.

Die Planungseinheit umfasst vorzugsweise eine Kommunikationseinheit, welche Kommunikationseinheit dazu ausgebildet, eine Information hinsichtlich der Zuweisung der flexiblen Gradientenverstärkereinheit zu dem ersten Zeitpunkt und/oder zu dem zweiten Zeitpunkt an das erste Magnetresonanzgerät und/oder das zweite Magnetresonanzgerät und/oder die erste Gradientenspuleneinheit und/oder die zweite Gradientenspuleneinheit und/oder die Steuereinheit zu übermitteln. Insbesondere umfasst die Information hinsichtlich der Zuweisung eine Eigenschaft umfassend eine Verfügbarkeit der flexiblen Gradientenverstärkereinheit zu einem Zeitpunkt.

Die Berücksichtigung der ersten Ansteuerungsinformation und/oder der zweiten Ansteuerungsinformation ermöglicht eine effiziente Verwendung nur einer Gradientenverstärkereinheit, der flexiblen Gradientenverstärkereinheit unter besonders guter Berücksichtigung der Anforderungen des Gradientensystems. Diese Ausführungsform ermöglicht demnach einen kontinuierlichen Betrieb beider Gradientenspuleneinheiten, insbesondere beider Magnetresonanzsysteme.

Eine Ausführungsform des Gradientensystems sieht vor, dass die Planungseinheit dazu ausgebildet ist, eine Ansteuerung der ersten Gradientenspuleneinheit und/oder der zweiten Gradientenspuleneinheit durch die flexible Gradientenverstärkereinheit gemäß der Zuweisung zu initiieren. Dies ermöglicht eine effiziente Realisierung der geplanten Verwendung der flexiblen Gradientenverstärkereinheit.

Eine Ausführungsform des Gradientensystems sieht vor, dass die Planungseinheit eine Prüfeinheit umfasst, welche Prüfeinheit dazu ausgebildet ist, zumindest eine erste Spezifikation für die erste Gradientenspuleneinheit
zu erfassen, und sicherzustellen, dass bei Ansteuerung der ersten Gradientenspuleneinheit durch die flexible Gradientenverstärkereinheit die erste Spezifikation eingehalten wird. Die erste Spezifikation kann der Planungseinheit, insbesondere der Prüfeinheit, über eine von der Planungseinheit umfasste Kommunikationseinheit und/oder einen von der Planungseinheit umfassten Planungseingang bereitgestellt werden.

Eine Gradientenverstärkereinheit induziert typischerweise einen elektrischen Strom und/oder eine elektrische Spannung innerhalb einer Gradientenspuleneinheit zur Erzeugung eines Magnetfeldgradienten. Abhängig von der Ausführungsform der Gradientenspuleneinheit ist typischerweise ein maximaler elektrischer Strom und/oder eine maximale elektrische Spannung zulässig. Die erste Spezifikation umfasst typischerweise einen maximal zulässigen elektrischen Strom und/oder eine maximal zulässige elektrische Spannung und/oder eine maximale Anstiegs- und Abfallrate für die ersten Gradientenspuleneinheit.

Die flexible Gradientenverstärkereinheit kann dazu ausgebildet sein, die erste Gradientenspuleneinheit außerhalb der ersten Spezifikation zu betreiben. Dies kann die erste Gradientenspuleneinheit jedoch beschädigen. Die Prüfeinheit kann demnach sicherstellen, dass ein von der flexiblen Gradientenverstärkereinheit an die erste Gradientenspuleneinheit ausgegebener elektrischer Strom und/oder elektrische Spannung innerhalb der ersten Spezifikation liegt.

Diese Ausführungsform verhindert eine Beschädigung der ersten Gradientenspuleneinheit durch eine Ansteuerung durch die flexible Gradientenverstärkereinheit.

Eine Ausführungsform des Gradientensystems sieht vor, dass die erste Gradientenspuleneinheit und die zweite Gradientenspuleneinheit innerhalb eines Gebäudes angeordnet sind. Insbesondere ist das Gradientensystem innerhalb eines Gebäudes angeordnet. Vorzugsweise ist das Gradientensystem innerhalb eines klinischen Betriebes angeordnet. Der räumliche Abstand zwischen der ersten Gradientenspuleneinheit und der zweiten Gradientenspuleneinheit beträgt typischerweise weniger als 250 m, vorzugsweise weniger als 100 m, besonders bevorzugt weniger als 50 m. Die erste Gradientenspuleneinheit, die zweite Gradientenspuleneinheit und die flexible Gradientenverstärkereinheit sind vorzugsweise in voneinander verschiedenen Räumen angeordnet. Die erste Gradientenspuleneinheit, die zweite Gradientenspuleneinheit und die flexible Gradientenverstärkereinheit sind vorzugsweise in zumindest teilweise aneinander angrenzenden Räumen angeordnet.

Hierdurch können mehrere, innerhalb eines klinischen Betriebes verwendete Magnetresonanzgeräte und/oder Gradientenspuleneinheiten von der einen flexibel verwendbaren Gradientenverstärkereinheit profitieren. Hierdurch können die Magnetresonanzgeräte und/oder Gradientenspuleneinheiten besonders kostengünstig betrieben werden.

Eine Ausführungsform des Gradientensystems sieht vor, dass das Gradientensystem eine erste statische Gradientenverstärkereinheit und eine zweite statische Gradientenverstärkereinheit umfasst und
die erste statische Gradientenverstärkereinheit der ersten Gradientenspuleneinheit und die zweite statische Gradientenverstärkereinheit der zweiten Gradientenspuleneinheit zugeordnet ist.

Herkömmlich ist einer Gradientenspuleneinheit genau eine statische Gradientenverstärkereinheit zugewiesen. Die erste statische Gradientenverstärkereinheit ist im Rahmen dieser Ausführungsform des Gradientensystems typischerweise derart mit der ersten Gradientenspuleneinheit verbunden, dass die erste statische Gradientenverstärkereinheit nur zur Ansteuerung der ersten Gradientenspuleneinheit ausgebildet ist. Die erste statische Gradientenverstärkereinheit ist typischerweise nur dann zur Ansteuerung einer anderen Gradientenspuleneinheit als der ersten Gradientenspuleneinheit ausgebildet, wenn die erste statische Gradientenverstärkereinheit manuell, typischerweise von einer technisch ausgebildeten Fachkraft, mit der anderen Gradientenspuleneinheit als der ersten Gradientenspuleneinheit verbunden wird.
Die zweite statische Gradientenverstärkereinheit ist im Rahmen dieser Ausführungsform des Gradientensystems typischerweise derart mit der zweiten Gradientenspuleneinheit verbunden, dass die zweite statische Gradientenverstärkereinheit nur zur Ansteuerung der zweiten Gradientenspuleneinheit ausgebildet ist. Die zweite statische Gradientenverstärkereinheit ist typischerweise nur dann zur Ansteuerung einer anderen Gradientenspuleneinheit als der zweiten Gradientenspuleneinheit ausgebildet, wenn die erste statische Gradientenverstärkereinheit manuell, typischerweise von einer technisch ausgebildeten Fachkraft, mit der anderen Gradientenspuleneinheit als der zweiten Gradientenspuleneinheit verbunden wird. Die erste statische Gradientenverstärkereinheit und/oder die zweite statische Gradientenverstärkereinheit kann einer herkömmlichen Gradientenverstärkereinheit entsprechen. Insbesondere umfassen die erste statische Gradientenverstärkereinheit und/oder die zweite statische Gradientenverstärkereinheit Schnittstellen, welche eine Datenübertragung mit einer Steuereinheit und/oder der flexiblen Schnittstelle ermöglichen.

Diese Ausführungsform ermöglicht eine Ansteuerung der ersten Gradientenspuleneinheit durch die erste statische Gradientenverstärkereinheit und/oder die flexible Gradientenverstärkereinheit.

Diese Ausführungsform ermöglicht eine Ansteuerung der zweiten Gradientenspuleneinheit durch die zweite statische Gradientenverstärkereinheit und/oder die flexible Gradientenverstärkereinheit. Dies ermöglicht eine Verwendung der flexiblen Gradientenverstärkereinheit abhängig von einer Art der Ansteuerung der ersten Gradientenspuleneinheit und/oder der zweiten Gradientenspuleneinheit. Insbesondere kann abhängig von einer erforderlichen Leistung die flexible Gradientenverstärkereinheit verwendet werden. Dadurch kann ein Gradientensystem besonders kostengünstig ausgestaltet sein.

Eine Ausführungsform des Gradientensystems sieht vor, dass eine durch die flexible Gradientenverstärkereinheit maximal erzeugbare Leistung größer als eine durch die erste statische Gradientenverstärkereinheit maximal erzeugbare Leistung ist und dass eine durch die durch die flexible Gradientenverstärkereinheit maximal erzeugbare Leistung größer als eine durch die zweite statische Gradientenverstärkereinheit maximal erzeugbare Leistung ist.

Die maximal erzeugbare Leistung einer Gradientenverstärkereinheit kann beispielsweise ein Maß für die maximale elektrische Spannung und/oder die maximale elektrische Stromstärke in einer mit der Gradientenverstärkereinheit verbundenen Gradientenspuleneinheit sein. Die maximal erzeugbare Leistung einer Gradientenverstärkereinheit kann beispielsweise ein Maß für die maximale Anstiegs- und Abfallrate eines von einer mit der Gradientenverstärkereinheit verbundenen Gradientenspuleneinheit erzeugten Magnetfeldgradienten sein.

Eine Leistung kann beispielsweise ein Maß für eine elektrische Spannung und/oder eine elektrische Stromstärke in einer Gradientenspuleneinheit und/oder eine Anstiegs- und Abfallrate eines von einer Gradientenspuleneinheit erzeugten Magnetfeldgradienten sein. Es wurde erkannt, dass bei etwa 90% der im klinischen Betrieb ausgeführten Untersuchungen und/oder MR-Steuerungssequenzen eine Leistung benötigt wird, die weniger als 50%, typischerweise weniger als 30% und/oder 20% der Maximalleistung beträgt. Die Maximalleistung kann dadurch definiert sein, dass diese die maximale für eine Gradientenspuleneinheit zulässige Leistung ist. Herkömmlich ist eine statische Gradientenverstärkereinheit, welche einer Gradientenspuleneinheit direkt zugeordnet ist, zur Erzeugung dieser Maximalleistung ausgelegt.

MR-Steuerungssequenzen, welche häufig verwendet werden, jedoch typischerweise weniger als 30% der Maximalleistung erfordern, sind beispielsweise spinecho-basierte MR-Steuerungssequenzen. Eine MR-Steuerungssequenzen, welche beispielsweise zumindest 80% der Maximalleistung erfordert, ist eine EPI.

Diese Ausführungsform sieht vor, dass die erste statische Gradientenverstärkereinheit und die zweite statische Gradientenverstärkereinheit zu einer geringeren maximalen Leistung ausgebildet sind als die flexible Gradientenverstärkereinheit. Die maximal erzeugbare Leistung der ersten statischen Gradientenverstärkereinheit und/oder der zweiten statischen Gradientenverstärkereinheit beträgt typischerweise weniger als 80%, vorzugsweise weniger als 60%, besonders bevorzugt weniger als 40% der Maximalleistung der ersten Gradientenspuleneinheit und/oder zweiten Gradientenspuleneinheit.

Für eine Ansteuerung der ersten Gradientenspuleneinheit und/oder der zweiten Gradientenspuleneinheit mit einer Leistung größer als der maximal erzeugbaren Leistung der ersten statischen Gradientenverstärkereinheit und/oder zweiten statischen Gradientenverstärkereinheit ist demnach die flexible Gradientenverstärkereinheit erforderlich.

Diese Ausführungsform ermöglicht eine kontinuierliche Nutzung aller vom Gradientensystem umfassten Gradientenspuleneinheiten mit niedriger Leistung. Falls eine Ansteuerung einer Gradientenspuleneinheit mit hoher Leistung, beispielsweise im Rahmen einer EPI, geplant ist, so wird die flexible Gradientenverstärkereinheit zur Ansteuerung dieser Gradientenspuleneinheit verwendet.

Hierdurch kann eine teure Komponente, wie die flexible Gradientenverstärkereinheit, besser ausgelastet werden. Die statischen Gradientenverstärkereinheiten können kostengünstig hergestellt werden, wodurch sich der Gesamtpreis des Gradientensystems, insbesondere bei einer höheren Zahl an Gradientenspuleneinheiten. Insbesondere bei Kombination dieser Ausführungsform mit der Planungseinheit kann eine besonders effiziente Ansteuerung der statischen Gradientenverstärkereinheiten und der flexiblen Gradientenverstärkereinheit erfolgen. Zudem kann die flexible Gradientenverstärkereinheit in bestehenden Gradientensystemen nachgerüstet werden. Die flexible Gradientenverstärkereinheit ist leicht austauschbar und kann insbesondere gegen eine neue, leistungsstärkere flexible Gradientenverstärkereinheit bei Bedarf ausgetauscht werden.

Ebenso kann eine Gradientenspuleneinheit unabhängig von der zugeordneten statischen Gradientenverstärkereinheit ausgelegt werden, insbesondere für eine höhere Leistung. Dadurch kann das der Gradientenspuleneinheit zugeordnete Magnetresonanzgerät verbessert werden, beispielsweise hinsichtlich von diesem erzeugten Bilddaten.

Das erfindungsgemäße Bildgebungssystem umfasst ein Gradientensystem, ein erstes Magnetresonanzgerät und ein zweites Magnetresonanzgerät, wobei der ersten Gradientenspuleneinheit das erste Magnetresonanzgerät und der zweiten Gradientenspuleneinheit das zweite Magnetresonanzgerät zugeordnet ist.

Das erste Magnetresonanzgerät und das zweite Magnetresonanzgerät sind vorzugsweise mit einer von der Planungseinheit umfassten Kommunikationseinheit verbunden.

Eine allgemeine Funktionsweise eines Magnetresonanzgeräts, also des ersten Magnetresonanzgeräts und des zweiten Magnetresonanzgeräts, ist dem Fachmann bekannt, so dass auf eine detaillierte Beschreibung der weiteren Komponenten verzichtet wird. Das erste Magnetresonanzgerät umfasst typischerweise die erste Gradientenspuleneinheit. Das zweite Magnetresonanzgerät umfasst typischerweise die zweite Gradientenspuleneinheit.

Ausführungsformen des erfindungsgemäßen Bildgebungssystems sind analog zu den Ausführungsformen des Gradientensystems ausgebildet.

Das erfindungsgemäße Verfahren zu einer Ansteuerung eines von einem erfindungsgemäßen Bildgebungssystem umfassten Gradientensystems zusätzlich umfassend eine erste statische Gradientenverstärkereinheit und eine zweite statische Gradientenverstärkereinheit, wobei die erste statische Gradientenverstärkereinheit der ersten Gradientenspuleneinheit und die zweite statische Gradientenverstärkereinheit der zweiten Gradientenspuleneinheit zugeordnet ist, sieht die folgenden Verfahrensschritte vor:
- Bereitstellen einer ersten Ansteuerungsinformation über eine Ansteuerung der ersten Gradientenspuleneinheit zu einem ersten Zeitpunkt,
- Ermitteln einer ersten erforderlichen Leistung für die erste Gradientenspuleneinheit basierend auf der ersten Ansteuerungsinformation,
- Analyse umfassend einen ersten Vergleich der ersten erforderlichen Leistung mit einer durch die erste statische Gradientenverstärkereinheit maximal erzeugbaren Leistung,
- Zuweisung der flexiblen Gradientenverstärkereinheit an die erste Gradientenspuleneinheit und/oder an die zweite Gradientenspuleneinheit und/oder in eine Ruheposition abhängig von der Analyse.

Die Zuweisung abhängig von der Analyse sieht typischerweise vor, dass bei einem Überschreiten der durch die erste statische Gradientenverstärkereinheit maximal erzeugbaren Leistung von der ersten erforderlichen Leistung, die flexible Gradientenverstärkereinheit an die erste Gradientenspuleneinheit zur Ansteuerung der ersten Gradientenspuleneinheit gemäß der ersten Ansteuerungsinformation zugewiesen wird.

Die Zuweisung abhängig von der Analyse sieht typischerweise vor, dass bei einem Unterschreiten der durch die erste statische Gradientenverstärkereinheit maximal erzeugbaren Leistung von der ersten erforderlichen Leistung, die erste statische Gradientenverstärkereinheit an die erste Gradientenspuleneinheit zur Ansteuerung der ersten Gradientenspuleneinheit gemäß der ersten Ansteuerungsinformation zugewiesen wird und/oder die flexible Gradientenverstärkereinheit in eine Ruheposition zugewiesen wird.

Die Zuweisung abhängig von der Analyse erfolgt typischerweise zeitabhängig und/oder spezifisch für den ersten Zeitpunkt.

Die Analyse kann zusätzlich einen dritten Vergleich der ersten erforderlichen Leistung mit einer durch die flexible Gradientenverstärkereinheit maximal erzeugbare Leistung umfassen. Bei einem Überschreiten der durch die flexible Gradientenverstärkereinheit maximal erzeugbaren Leistung von der ersten erforderlichen Leistung kann die flexible Gradientenverstärkereinheit in eine Ruheposition zugewiesen werden.

Das Verfahren sieht vorzugsweise als weiteren Verfahrensschritt vor, dass eine Information hinsichtlich der Zuweisung und/oder Verwendung der flexiblen Gradientenverstärkereinheit zum ersten Zeitpunkt an eine Steuereinheit und/oder das erste Magnetresonanzgerät und/oder das zweite Magnetresonanzgerät bereitgestellt wird. Diese Information kann auch eine Reservierung der flexiblen Gradientenverstärkereinheit zum ersten Zeitpunkt für die erste Gradientenspuleneinheit umfassen.

Das Verfahren ermöglicht eine besonders effiziente Nutzung eines erfindungsgemäßen Gradientensystems. Insbesondere kann abhängig von der ersten Ansteuerungsinformation für die erste Gradientenspuleneinheit die richtige Gradientenverstärkereinheit, insbesondere die flexible Gradientenverstärkereinheit oder die erste statische Gradientenverstärkereinheit gewählt und zur Ansteuerung des ersten Magnetresonanzgerätes und/oder der ersten Gradientenspuleneinheit verwendet werden.

Eine Ausführungsform des Verfahrens sieht vor, dass die Zuweisung eine zeitliche Verschiebung des ersten Zeitpunktes umfasst. Die Analyse umfasst vorzugsweise eine Prüfung der Verfügbarkeit der flexiblen Gradientenverstärkereinheit zum ersten Zeitpunkt. Zeigt der Vergleich ein Überschreiten der durch die erste statische Gradientenverstärkereinheit maximal erzeugbaren Leistung von der ersten erforderlichen Leistung und zeigt die Prüfung, dass die flexible Gradientenverstärkereinheit zum ersten Zeitpunkt nicht verfügbar ist, so kann der erste Zeitpunkt, insbesondere eine Ansteuerung der ersten Gradientenspuleneinheit gemäß der ersten Ansteuerungsinformation zeitlich verschoben werden, die Ansteuerung der ersten Gradientenspuleneinheit gemäß der ersten Ansteuerungsinformation kann demnach zu einem anderen als dem ersten Zeitpunkt erfolgen. Alternativ kann die Ansteuerung der ersten Gradientenspuleneinheit zum ersten Zeitpunkt gemäß einer weiteren, von der ersten Ansteuerungsinformation verschiedenen Ansteuerungsinformation, erfolgen. Diese Ausführungsform ermöglicht eine flexible und adaptive Nutzung der flexiblen Gradientenverstärkereinheit.

Eine Ausführungsform des Verfahrens sieht vor, dass das Verfahren zusätzlich die folgenden Verfahrensschritte umfasst:
- Bereitstellen einer zweiten Ansteuerungsinformation über eine Ansteuerung der zweiten Gradientenspuleneinheit zu einem zweiten Zeitpunkt
- Ermitteln einer zweiten erforderlichen Leistung für die zweite Gradientenspuleneinheit basierend auf der zweiten Ansteuerungsinformation
und die Analyse einen zweiten Vergleich der zweiten erforderlichen Leistung mit einer durch die zweite statische Gradientenverstärkereinheit maximal erzeugbare Leistung umfasst und unter Berücksichtigung des ersten Zeitpunktes und des zweiten Zeitpunktes erfolgt.

Übersteigt beispielsweise die zweite erforderliche Leistung die durch die zweite statische Gradientenverstärkereinheit maximal erzeugbare Leistung und untersteigt die erste erforderliche Leistung die durch die erste statische Gradientenverstärkereinheit maximal erzeugbare Leistung, so kann die flexible Gradientenverstärkereinheit der zweiten Gradientenspuleneinheit zum zweiten Zeitpunkt zugewiesen werden. Übersteigt beispielsweise die zweite erforderliche Leistung die durch die zweite statische Gradientenverstärkereinheit maximal erzeugbare Leistung und übersteigt die erste erforderliche Leistung die durch die erste statische Gradientenverstärkereinheit maximal erzeugbare Leistung, so kann die flexible Gradientenverstärkereinheit abhängig vom ersten Zeitpunkt und zweiten Zeitpunkt der zweiten Gradientenspuleneinheit und/oder der ersten Gradientenspuleneinheit zugewiesen werden. Dies ermöglicht eine besonders effiziente Nutzung der flexiblen Gradientenverstärkereinheit.

Eine Ausführungsform des Verfahrens sieht vor, dass die Zuweisung eine zeitliche Verschiebung des zweiten Zeitpunktes umfasst. Analog zum ersten Zeitpunkt kann auch der zweite Zeitpunkt verschoben werden.

Ein erfindungsgemäßes Computerprogrammprodukt ist direkt in einer Speichereinheit der programmierbaren Planungseinheit ladbar und weist Programmcode-Mittel auf, um ein erfindungsgemäßes Verfahren auszuführen, wenn das Computerprogrammprodukt in der Planungseinheit ausgeführt wird. Dadurch kann das erfindungsgemäße Verfahren schnell, identisch wiederholbar und robust ausgeführt werden. Das Computerprogrammprodukt ist so konfiguriert, dass es mittels der Planungseinheit die erfindungsgemäßen Verfahrensschritte ausführen kann. Die Planungseinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem elektronisch lesbaren Medium gespeichert oder auf einem Netzwerk oder Server hinterlegt, von wo es in den Prozessor einer lokalen Planungseinheit geladen werden kann, der mit dem Gradientensystem direkt verbunden oder als Teil des Gradientensystems ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Planungseinheit eines Gradientensystems ein erfindungsgemäßes Verfahren durchführen. Beispiele für elektronisch lesbare Datenträger sind eine DVD, ein Magnetband oder einen USB-Stick, auf welchem elektronisch lesbare Steuerinformationen, insbesondere Software, gespeichert ist. Wenn diese Steuerinformationen (Software) von dem Datenträger gelesen und in eine Steuerungseinheit und/oder Planungseinheit eines Gradientensystems gespeichert werden, können alle erfindungsgemäßen Ausführungsformen der vorab beschriebenen Verfahren durchgeführt werden.

Des Weiteren geht die Erfindung aus von einem elektronisch lesbaren Datenträger, auf dem ein Programm hinterlegt ist, das zu einer Ausführung eines Verfahrens zu einer Ansteuerung eines erfindungsgemäßen Bildgebungssystems zusätzlich umfassend eine erste statische Gradientenverstärkereinheit und eine zweite statische Gradientenverstärkereinheit, wobei die erste statische Gradientenverstärkereinheit der ersten Gradientenspuleneinheit und die zweite statische Gradientenverstärkereinheit der zweiten Gradientenspuleneinheit zugeordnet ist, vorgesehen ist.

Die Vorteile des erfindungsgemäßen Bildgebungssystems, des erfindungsgemäßen Verfahrens, des erfindungsgemäßen Computerprogrammprodukts und des erfindungsgemäßen elektronisch lesbaren Datenträgers entsprechen im Wesentlichen den Vorteilen des Gradientensystems, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Weitere Vorteile, Merkmale und Einzelheiten der Erfindung ergeben sich aus den im Folgenden beschriebenen Ausführungsbeispielen sowie anhand der Zeichnungen.

Es zeigen:
- Fig. 1: eine schematische Darstellung einer ersten Ausführungsform eines Gradientensystems,
- Fig. 2: eine schematische Darstellung einer zweiten Ausführungsform eines Gradientensystems,
- Fig. 3: eine schematische Darstellung einer Ausführungsform eines erfindungsgemäßen Bildgebungssystem, und
- Fig. 4: ein Ablaufdiagramm einer Ausführungsform eines erfindungsgemäßen Verfahrens.

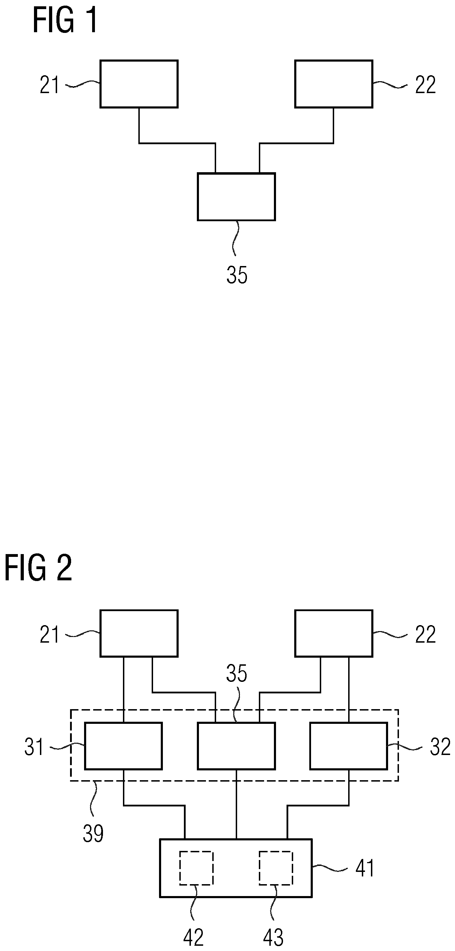

Figur 1 zeigt eine schematische Darstellung einer ersten Ausführungsform eines Gradientensystems. Das Gradientensystem umfasst eine erste Gradientenspuleneinheit 21, eine zweite Gradientenspuleneinheit 22 und eine flexible Gradientenverstärkereinheit 35, wobei die flexible Gradientenverstärkereinheit 35 zu einer Ansteuerung der ersten Gradientenspuleneinheit 21 und der zweiten Gradientenspuleneinheit 22 ausgebildet ist.

Figur 2 zeigt eine schematische Darstellung einer zweiten Ausführungsform eines Gradientensystems.

Das Gradientensystem gemäß dieser Ausführungsform umfasst eine erste statische Gradientenverstärkereinheit 31 und eine zweite statische Gradientenverstärkereinheit 32. Die erste statische Gradientenverstärkereinheit 31 ist der ersten Gradientenspuleneinheit 21 zugeordnet. Die zweite statische Gradientenverstärkereinheit 32 ist der zweiten Gradientenspuleneinheit 22 zugeordnet. Eine durch die flexible Gradientenverstärkereinheit 35 maximal erzeugbare Leistung ist größer als eine durch die erste statische Gradientenverstärkereinheit 31 maximal erzeugbare Leistung. Eine durch die flexible Gradientenverstärkereinheit 35 maximal erzeugbare Leistung ist größer als eine durch die zweite statische Gradientenverstärkereinheit 32 maximal erzeugbare Leistung. Die erste statische Gradientenverstärkereinheit 31, die zweite statische Gradientenverstärkereinheit 32 und die flexible Gradientenverstärkereinheit 35 bilden zusammen die Gesamtgradientenverstärkereinheit 39.

Das Gradientensystem umfasst zusätzlich eine Planungseinheit 41 umfassend einen Planungseingang 42. Der Planungseingang 42 ist dazu ausgebildet, eine erste Ansteuerungsinformation über eine Ansteuerung der ersten Gradientenspuleneinheit 21 zu einem ersten Zeitpunkt und/oder eine zweite Ansteuerungsinformation über eine Ansteuerung der zweiten Gradientenspuleneinheit 22 zu einem zweiten Zeitpunkt zu erfassen. Die Planungseinheit 41 ist dazu ausgebildet, basierend auf der ersten Ansteuerungsinformation und/oder der zweiten Ansteuerungsinformation eine Zuweisung der flexiblen Gradientenverstärkereinheit 35 zu dem ersten Zeitpunkt und/oder zu dem zweiten Zeitpunkt an die erste Gradientenspuleneinheit 21 und/oder an die zweite Gradientenspuleneinheit 22 vorzunehmen. Die Planungseinheit 41 ist vorzugsweise mit der ersten statischen Gradientenverstärkereinheit 31, der zweiten statischen Gradientenverstärkereinheit 32 und der flexiblen Gradientenverstärkereinheit 35 verbunden. Die Planungseinheit 41 ist vorzugsweise dazu ausgebildet, abhängig von der ersten Ansteuerungsinformation und/oder der zweiten Ansteuerungsinformation eine Ansteuerung der ersten Gradientenspuleneinheit 21 zum ersten Zeitpunkt durch die erste statische Gradientenverstärkereinheit 31 oder die flexible Gradientenverstärkereinheit 35 zu initiieren.

Die Planungseinheit 41 ist vorzugsweise dazu ausgebildet, abhängig von der ersten Ansteuerungsinformation und/oder der zweiten Ansteuerungsinformation eine Ansteuerung der zweiten Gradientenspuleneinheit 22 zum zweiten Zeitpunkt durch die zweite statische Gradientenverstärkereinheit 32 oder die flexible Gradientenverstärkereinheit 35 zu initiieren.

Die Planungseinheit 41 ist vorzugsweise dazu ausgebildet, abhängig von der ersten Ansteuerungsinformation und/oder der zweiten Ansteuerungsinformation und/oder dem ersten Zeitpunkt und/oder dem zweiten Zeitpunkt eine Ansteuerung der ersten Gradientenspuleneinheit 21 zu einem anderen Zeitpunkt als dem ersten Zeitpunkt und/oder der zweiten Gradientenspuleneinheit 22 zu einem anderen Zeitpunkt als dem zweiten Zeitpunkt zu initiieren.

Optional kann die Planungseinheit 41 eine Prüfeinheit 43 umfassen, welche Prüfeinheit 43 dazu ausgebildet ist, zumindest eine erste Spezifikation für die erste Gradientenspuleneinheit 21 zu erfassen, und sicherzustellen, dass bei Ansteuerung der ersten Gradientenspuleneinheit durch die flexible Gradientenverstärkereinheit die erste Spezifikation eingehalten wird. Die Prüfeinheit 43 ist vorzugsweise auch dazu ausgebildet, eine zweite Spezifikation für die zweite Gradientenspuleneinheit 22 zu erfassen, und sicherzustellen, dass bei Ansteuerung der zweiten Gradientenspuleneinheit 22 durch die flexible Gradientenverstärkereinheit 35 die zweite Spezifikation eingehalten wird.

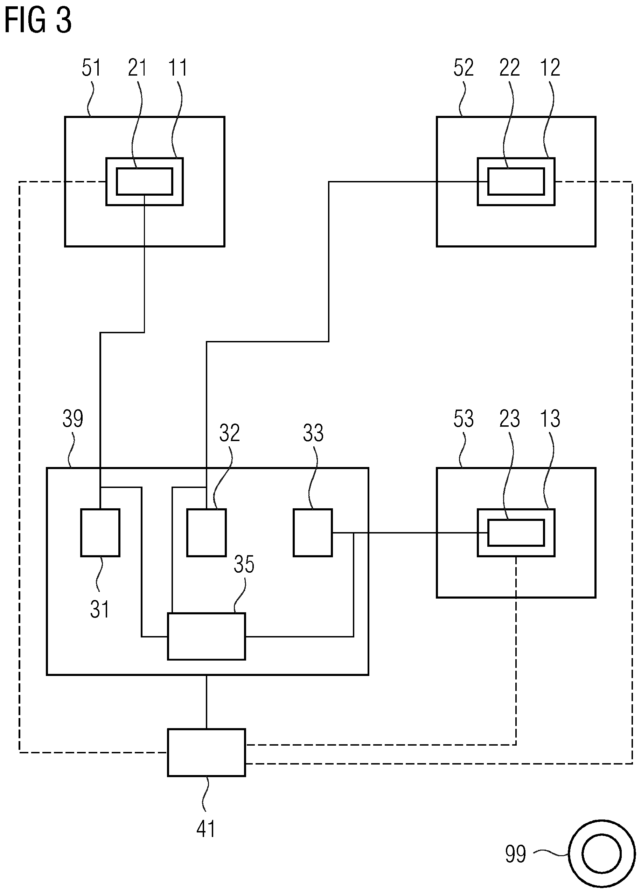

Figur 3 zeigt eine schematische Darstellung einer Ausführungsform eines erfindungsgemäßen Bildgebungssystems.

Das Bildgebungssystem umfasst ein erstes Magnetresonanzgerät 11 umfassend eine erste Gradientenspuleneinheit 21, welches innerhalb eines ersten Raumes 51 angeordnet ist.

Das Bildgebungssystem umfasst ein zweites Magnetresonanzgerät 12 umfassend eine zweite Gradientenspuleneinheit 22, welches innerhalb eines zweiten Raumes 52 angeordnet ist.

Das Bildgebungssystem umfasst ein drittes Magnetresonanzgerät 13 umfassend eine dritte Gradientenspuleneinheit 23, welches innerhalb eines dritten Raumes 53 angeordnet ist. Der erste Raum 51, der zweite Raum 52 und/oder der dritte Raum 53 sind vorzugsweise jeweils HF-abgeschirmte Räume.

Dem ersten Magnetresonanzgerät 11 ist die erste statische Gradientenverstärkereinheit 31 zugeordnet. Die erste Gradientenspuleneinheit 21 ist mit der ersten statischen Gradientenverstärkereinheit 31 verbunden, vorzugsweise über eine erste Leistungsschnittstelle. Dem zweiten Magnetresonanzgerät 12 ist die zweite statische Gradientenverstärkereinheit 32 zugeordnet. Die zweite Gradientenspuleneinheit 22 ist mit der zweiten statischen Gradientenverstärkereinheit 32 verbunden, vorzugsweise über eine zweite Leistungsschnittstelle. Dem dritten Magnetresonanzgerät 13 ist die dritte statische Gradientenverstärkereinheit 33 zugeordnet. Die dritte Gradientenspuleneinheit 23 ist mit der dritten statischen Gradientenverstärkereinheit 33 verbunden, vorzugsweise über eine dritte Leistungsschnittstelle.

Die flexible Gradientenverstärkereinheit 35 ist mit dem ersten Magnetresonanzgerät 11, insbesondere mit der ersten Gradientenspuleneinheit 21 verbunden. Hierfür kann die flexible Gradientenverstärkereinheit 35 eine eigene Verbindung mit der ersten Gradientenspuleneinheit 21 aufweisen. Die flexible Gradientenverstärkereinheit 35 kann auch an die erste Leistungsschnittstelle ankoppeln. Hierdurch können beispielsweise Leitungen, insbesondere Kabel, welche die erste Gradientenspuleneinheit 21 mit der ersten statischen Gradientenverstärkereinheit 31 verbinden, zumindest teilweise für eine Verbindung zwischen der ersten Gradientenspuleneinheit 21 und der flexiblen Gradientenverstärkereinheit 35 verwendet werden.

Die flexible Gradientenverstärkereinheit 35 ist mit dem zweiten Magnetresonanzgerät 12, insbesondere mit der zweiten Gradientenspuleneinheit 22 verbunden. Hierfür kann die flexible Gradientenverstärkereinheit 35 eine eigene Verbindung mit der zweiten Gradientenspuleneinheit 22 aufweisen. Die flexible Gradientenverstärkereinheit 35 kann auch an die zweite Leistungsschnittstelle ankoppeln. Hierdurch können beispielsweise Leitungen, insbesondere Kabel, welche die zweite Gradientenspuleneinheit 22 mit der zweiten statischen Gradientenverstärkereinheit 32 verbinden, zumindest teilweise für eine Verbindung zwischen der zweiten Gradientenspuleneinheit 22 und der flexiblen Gradientenverstärkereinheit 35 verwendet werden.

Die flexible Gradientenverstärkereinheit 35 ist mit dem dritten Magnetresonanzgerät 13, insbesondere mit der dritten Gradientenspuleneinheit 23 verbunden. Hierfür kann die flexible Gradientenverstärkereinheit 35 eine eigene Verbindung mit der dritten Gradientenspuleneinheit 23 aufweisen. Die flexible Gradientenverstärkereinheit 35 kann auch an die dritte Leistungsschnittstelle ankoppeln. Hierdurch können beispielsweise Leitungen, insbesondere Kabel, welche die dritte Gradientenspuleneinheit 23 mit der dritten statischen Gradientenverstärkereinheit 33 verbinden, zumindest teilweise für eine Verbindung zwischen der dritten Gradientenspuleneinheit 23 und der flexiblen Gradientenverstärkereinheit 35 verwendet werden.

Die erste statische Gradientenverstärkereinheit 31, die zweite statische Gradientenverstärkereinheit 32, die dritte statische Gradientenverstärkereinheit 33 und die flexible Gradientenverstärkereinheit 35 bilden die Gesamtgradientenverstärkereinheit 39. Die Gesamtgradientenverstärkereinheit 39 ist mit der Planungseinheit 41. Die Planungseinheit 41 kann analog zu der in Figur 2 dargestellten Planungseinheit ausgebildet sein.

Die Gesamtgradientenverstärkereinheit 39 ist vorzugsweise außerhalb des ersten Raumes 51 und/oder des zweiten Raumes 52 und/oder des dritten Raumes 53 angeordnet. Die Gesamtgradientenverstärkereinheit 39 ist vorzugsweise innerhalb eines Raumes angeordnet. Für den Betrieb der ersten statischen Gradientenverstärkereinheit 31, der zweiten statischen Gradientenverstärkereinheit 32, der dritten statischen Gradientenverstärkereinheit 33 und der flexiblen Gradientenverstärkereinheit 35 sind eine Kühlung und eine Versorgung mit Spannung erforderlich. Ist die Gesamtgradientenverstärkereinheit 39 innerhalb eines Raumes angeordnet, so kann die Versorgung mittels Kühlmittel und Spannung zentral erfolgen. Dies steigert die Effizienz der Gesamtgradientenverstärkereinheit 39 und vereinfacht deren Wartung. Die Planungseinheit 41 kann auch innerhalb dieses Raumes angeordnet sein. Der erste Raum 51 und/oder der zweite Raum 52 und/oder der dritte Raum 53 sind typischerweise innerhalb eines Gebäudes und/oder innerhalb eines klinischen Betriebes angeordnet.

Die Planungseinheit 41 ist dazu ausgebildet, erste Ansteuerungsinformation über eine Ansteuerung der ersten Gradientenspuleneinheit 21 zu einem ersten Zeitpunkt zu erfassen. Hierfür kann die Planungseinheit 41 mit dem ersten Magnetresonanzgerät 11 und/oder einer nicht näher dargestellten Steuereinheit für das erste Magnetresonanzgerät 11 verbunden sein. Die Planungseinheit 41 ist dazu ausgebildet, zweite Ansteuerungsinformation über eine Ansteuerung der zweiten Gradientenspuleneinheit 22 zu einem zweiten Zeitpunkt zu erfassen. Hierfür kann die Planungseinheit 41 mit dem zweiten Magnetresonanzgerät 12 und/oder einer nicht näher dargestellten Steuereinheit für das zweite Magnetresonanzgerät 12 verbunden sein. Die Planungseinheit 41 ist dazu ausgebildet, dritte Ansteuerungsinformation über eine Ansteuerung der dritten Gradientenspuleneinheit 23 zu einem dritten Zeitpunkt zu erfassen. Der dritte Zeitpunkt ist typischerweise ein zukünftiger Zeitpunkt. Hierfür kann die Planungseinheit 41 mit dem dritten Magnetresonanzgerät 13 und/oder einer nicht näher dargestellten Steuereinheit für das dritte Magnetresonanzgerät 13 verbunden sein.

Die Planungseinheit 41 ist dazu ausgebildet, abhängig von der ersten Ansteuerungsinformation und/oder zweiten Ansteuerungsinformation und/oder dritten Ansteuerungsinformation eine Ansteuerung des ersten Magnetresonanzgerätes 11 durch die erste statische Gradientenverstärkereinheit 31 oder die flexible Gradientenverstärkereinheit 35 zu veranlassen. Die Planungseinheit 41 ist dazu ausgebildet, abhängig von der ersten Ansteuerungsinformation und/oder zweiten Ansteuerungsinformation und/oder dritten Ansteuerungsinformation eine Ansteuerung des zweiten Magnetresonanzgerätes 12 durch die zweite statische Gradientenverstärkereinheit 32 oder die flexible Gradientenverstärkereinheit 35 zu veranlassen. Die Planungseinheit 41 ist dazu ausgebildet, abhängig von der ersten Ansteuerungsinformation und/oder zweiten Ansteuerungsinformation und/oder dritten Ansteuerungsinformation eine Ansteuerung des dritten Magnetresonanzgerätes 13 durch die dritte statische Gradientenverstärkereinheit 33 oder die flexible Gradientenverstärkereinheit 35 zu veranlassen.

Die Planungseinheit 41 ist dazu ausgebildet, abhängig von der ersten Ansteuerungsinformation und/oder zweiten Ansteuerungsinformation und/oder dritten Ansteuerungsinformation eine von der Gesamtgradientenverstärkereinheit 39 umfassten Gradientenverstärkereinheit zur Ansteuerung der ersten Gradientenspuleneinheit 21 und/oder der zweiten Gradientenspuleneinheit 22 und/oder der dritten Gradientenspuleneinheit 23 auszuwählen.

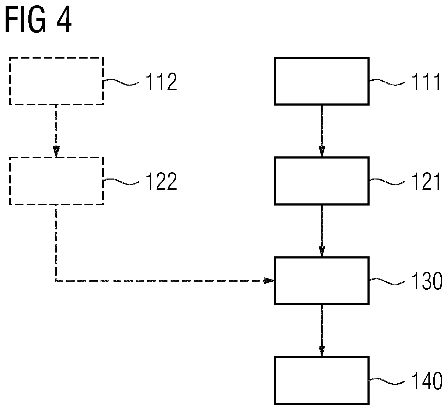

Figur 4 zeigt ein Ablaufdiagramm einer Ausführungsform eines erfindungsgemäßen Verfahrens zu einer Ansteuerung eines Gradientensystems.

Das Verfahren beginnt mit Verfahrensschritt 111, dem Bereitstellen einer ersten Ansteuerungsinformation über eine Ansteuerung der ersten Gradientenspuleneinheit 21 zu einem ersten Zeitpunkt. Im folgenden Verfahrensschritt 121 erfolgt die Ermittlung einer ersten erforderlichen Leistung für die erste Gradientenspuleneinheit 21 basierend auf der ersten Ansteuerungsinformation. In Verfahrensschritt 130 erfolgt eine Analyse umfassend einen ersten Vergleich der ersten erforderlichen Leistung mit einer durch die erste statische Gradientenverstärkereinheit 31 maximal erzeugbaren Leistung. Der folgende Verfahrensschritt 140 sieht abhängig von der Analyse eine Zuweisung der flexiblen Gradientenverstärkereinheit 35 an die erste Gradientenspuleneinheit 21 und/oder an die zweite Gradientenspuleneinheit 22 und/oder in eine Ruheposition vor.

Optional kann in Verfahrensschritt 112 eine Bereitstellung einer zweiten Ansteuerungsinformation über eine Ansteuerung der zweiten Gradientenspuleneinheit 22 zu einem zweiten Zeitpunkt erfolgen. Basierend auf der zweiten Ansteuerungsinformation erfolgt eine Ermittlung einer zweiten erforderlichen Leistung für die zweite Gradientenspuleneinheit 22 in Verfahrensschritt 122. Diese zweite erforderliche Leistung wird bei der Analyse im Rahmen des Verfahrensschritte 130 durch einen zweiten Vergleich der zweiten erforderlichen Leistung mit einer durch die zweite statische Gradientenverstärkereinheit 32 maximal erzeugbare Leistung berücksichtigt. Ebenso erfolgt die Analyse im Rahmen des Verfahrensschritte 130 unter Berücksichtigung des zweiten Zeitpunktes. Die Zuweisung der flexiblen Gradientenverstärkereinheit 35 an die erste Gradientenspuleneinheit 21 und/oder an die zweite Gradientenspuleneinheit 22 und/oder in eine Ruheposition kann auch eine zeitliche Verschiebung des ersten Zeitpunktes und/oder des zweiten Zeitpunktes umfassen.

Ein Verfahren zu einer Ansteuerung eines Gradientensystems kann auch in Form eines Computerprogrammprodukts vorliegen, dass das Verfahren auf die Planungseinheit 41 und/oder das Gradientensystem implementiert, wenn es auf der Planungseinheit 41 ausgeführt wird. Ebenso kann ein elektronisch lesbarer Datenträger 99 mit darauf gespeicherten elektronisch lesbaren Steuerinformationen vorliegen, welche zumindest ein solches eben beschriebenes Computerprogrammprodukt umfassen und derart ausgestaltet sind, dass sie bei Verwendung des Datenträgers 99 in einer Planungseinheit 41 das beschriebene Verfahren durchführen.

Obwohl die Erfindung im Detail durch die bevorzugten Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen, welcher durch die Ansprüche definiert ist.

## Patentansprüche

1. Bildgebungssystem umfassend ein erstes Magnetresonanzgerät (11), ein zweites Magnetresonanzgerät (12) und ein Gradientensystem umfassend eine erste Gradientenspuleneinheit (21), eine zweite Gradientenspuleneinheit (22) und eine flexible Gradientenverstärkereinheit (35) zur bedarfsgerechten Verwendung, wobei die flexible Gradientenverstärkereinheit (35) zu einer Ansteuerung der ersten Gradientenspuleneinheit (21) und der zweiten Gradientenspuleneinheit (22) ausgebildet ist, wobei der ersten Gradientenspuleneinheit (21) das erste Magnetresonanzgerät (11) und der zweiten Gradientenspuleneinheit (22) das zweite Magnetresonanzgerät (12) zugeordnet ist.

2. Bildgebungssystem nach Anspruch 1,
wobei das Gradientensystem eine Planungseinheit (41) umfassend einen Planungseingang (42) aufweist,
der Planungseingang (42) dazu ausgebildet ist, eine erste Ansteuerungsinformation über eine Ansteuerung der ersten Gradientenspuleneinheit (21) zu einem ersten Zeitpunkt und/oder eine zweite Ansteuerungsinformation über eine Ansteuerung der zweiten Gradientenspuleneinheit (22) zu einem zweiten Zeitpunkt zu erfassen,
und die Planungseinheit (41) dazu ausgebildet ist, basierend auf der ersten Ansteuerungsinformation und/oder der zweiten Ansteuerungsinformation eine Zuweisung der flexiblen Gradientenverstärkereinheit (35) zu dem ersten Zeitpunkt und/oder zu dem zweiten Zeitpunkt an die erste Gradientenspuleneinheit (21) und/oder an die zweite Gradientenspuleneinheit (22) vorzunehmen.

3. Bildgebungssystem nach Anspruch 2,
wobei die Planungseinheit (41) eine Prüfeinheit (43) umfasst, welche Prüfeinheit (43) dazu ausgebildet ist, zumindest eine erste Spezifikation für die erste Gradientenspuleneinheit (21) zu erfassen, und sicherzustellen, dass bei Ansteuerung der ersten Gradientenspuleneinheit (21) durch die flexible Gradientenverstärkereinheit (35) die erste Spezifikation eingehalten wird.

4. Bildgebungssystem nach einem der vorangehenden Ansprüche, wobei das Gradientensystem eine erste statische Gradientenverstärkereinheit (31), verbunden mit der ersten Gradientenspuleneinheit (21) und ausgebildet zur Ansteuerung nur der ersten Gradientenspuleneinheit (21), und eine zweite statische Gradientenverstärkereinheit (32), verbunden mit der zweiten Gradientenspuleneinheit (22) und ausgebildet zur Ansteuerung nur der zweiten Gradientenspuleneinheit (22), umfasst.

5. Bildgebungssystem nach Anspruch 4,
wobei eine durch die flexible Gradientenverstärkereinheit (35) maximal erzeugbare Leistung größer als eine durch die erste statische Gradientenverstärkereinheit (31) maximal erzeugbare Leistung und eine durch die flexible Gradientenverstärkereinheit (35) maximal erzeugbare Leistung größer als eine durch die zweite statische Gradientenverstärkereinheit (32) maximal erzeugbare Leistung ist.

6. Verfahren zu einer Ansteuerung eines von einem Bildgebungssystem nach einem der Ansprüche 4 bis 5 umfassten Gradientensystems gemäß den folgenden Verfahrensschritten
- Bereitstellen einer ersten Ansteuerungsinformation über eine Ansteuerung der ersten Gradientenspuleneinheit (21) zu einem ersten Zeitpunkt,
- Ermitteln einer ersten erforderlichen Leistung für die erste Gradientenspuleneinheit (21) basierend auf der ersten Ansteuerungsinformation,
- Analyse umfassend einen ersten Vergleich der ersten erforderlichen Leistung mit einer durch die erste statische Gradientenverstärkereinheit (31) maximal erzeugbaren Leistung,
- Zuweisung der flexiblen Gradientenverstärkereinheit (35) an die erste Gradientenspuleneinheit (21) und/oder an die zweite Gradientenspuleneinheit (22) und/oder in eine Ruheposition abhängig von der Analyse.

7. Verfahren nach Anspruch 6,
wobei die Zuweisung eine zeitliche Verschiebung des ersten Zeitpunktes umfasst.

8. Verfahren nach einem der Ansprüche 6 bis 7,
wobei das Verfahren zusätzlich die folgenden Verfahrensschritte umfasst:
- Bereitstellen einer zweiten Ansteuerungsinformation über eine Ansteuerung der zweiten Gradientenspuleneinheit (22) zu einem zweiten Zeitpunkt,
- Ermitteln einer zweiten erforderlichen Leistung für die zweite Gradientenspuleneinheit (22) basierend auf der zweiten Ansteuerungsinformation,
und die Analyse einen zweiten Vergleich der zweiten erforderlichen Leistung mit einer durch die zweite statische Gradientenverstärkereinheit (31) maximal erzeugbare Leistung umfasst und unter Berücksichtigung des ersten Zeitpunktes und des zweiten Zeitpunktes erfolgt.

9. Verfahren nach Anspruch 8,
wobei die Zuweisung eine zeitliche Verschiebung des zweiten Zeitpunktes umfasst.

10. Computerprogrammprodukt, welches ein Programm umfasst und direkt in einen Speicher einer von einem Bildgebungssystem nach einem der Ansprüche 4 bis 5 umfassten und programmierbaren Planungseinheit (41) ladbar ist, mit Programmmitteln, um ein Verfahren zu einer Ansteuerung eines vom Bildgebungssystem umfassten Gradientensystems nach einem der Ansprüche 6 bis 9 auszuführen, wenn das Programm in der Planungseinheit (41) ausgeführt wird.

11. Elektronisch lesbarer Datenträger (99), auf dem ein Programm hinterlegt ist, das derart ausgestaltet ist, dass das Programm bei Verwendung des Datenträgers (99) in einer von einem Bildgebungssystem nach einem der Ansprüche 4 bis 5 umfassten Planungseinheit (41) das Verfahren zu einer Ansteuerung des Bildgebungssystems nach einem der Ansprüche 6 bis 9 durchführt.

## Claims

1. Imaging system comprising a first magnetic resonance device (11), a second magnetic resonance device (12) and a gradient system comprising a first gradient coil unit (21), a second gradient coil unit (22) and a flexible gradient amplifier unit (35) to be used according to requirements, wherein the flexible gradient amplifier unit (35) is embodied to actuate the first gradient coil unit (21) and the second gradient coil unit (22), wherein the first magnetic resonance device (11) is assigned to the first gradient coil unit (21) and the second magnetic resonance device (12) is assigned to the second gradient coil unit (22).

2. Imaging system according to claim 1,
wherein the gradient system has a planning unit (41) comprising a planning input (42),
the planning input (42) is embodied to detect a first actuation information about an actuation of the first gradient coil unit (21) at a first point in time and/or a second actuation information about an actuation of the second gradient coil unit (22) at a second point in time,
and the planning unit (41) is embodied, based on the first actuation information and/or the second actuation information, to perform an assignment of the flexible gradient amplifier unit (35) at the first point in time and/or at the second point in time to the first gradient coil unit (21) and/or to the second gradient coil unit (22).

3. Imaging system according to claim 2,
wherein the planning unit (41) comprises a testing unit (43) which is embodied to detect at least a first specification for the first gradient coil unit (21), and to ensure compliance with the first specification upon actuation of the first gradient coil unit (21) by the flexible gradient amplifier unit (35).

4. Imaging system according to one of the preceding claims, wherein the gradient system comprises a first static gradient amplifier unit (31) connected to the first gradient coil unit (21) and embodied to actuate only the first gradient coil unit (21) and a second static gradient amplifier unit (32) connected to the second gradient coil unit (22) and embodied to actuate only the second gradient coil unit (22).

5. Imaging system according to claim 4,
wherein a maximum power which can be generated by the flexible gradient amplifier unit (35) is greater than a maximum power which can be generated by the first static gradient amplifier unit (31) and a maximum power which can be generated by the flexible gradient amplifier unit (35) is greater than a maximum power which can be generated by the second static gradient amplifier unit (32).

6. Method for actuating a gradient system comprised in an imaging system according to one of claims 4 to 5, according to the following method steps:
- Provision of a first actuation information about an actuation of the first gradient coil unit (21) at a first point in time,
- determination of a first required power for the first gradient coil unit (21) based on the first actuation information,
- analysis comprising a first comparison of the first required power with a maximum power which can be generated by the first static gradient amplifier unit (31) ,
- assignment of the flexible gradient amplifier unit (35) to the first gradient coil unit (21) and/or to the second gradient coil unit (22) and/or in an idle position depending on the analysis.

7. Method according to claim 6,
wherein the assignment comprises a temporal shift of the first point in time.

8. Method according to one of claims 6 to 7,
wherein the method also comprises the following method steps:
- Provision of a second actuation information about an actuation of the second gradient coil unit (22) at a second point in time
- Determination of a second required power for the second gradient coil unit (22) based on the second actuation information
and the analysis comprises a second comparison of the second required power with a maximum power which can be generated by the second static gradient amplifier unit (31) and takes place taking into consideration the first point in time and the second point in time.

9. Method according to claim 8,
wherein the assignment comprises a temporal shift of the second point in time.

10. Computer program product which comprises a program and is directly loadable into a memory store of a programmable planning unit (41) comprised in an imaging system according to one of claims 4 to 5, having program means in order to carry out a method for actuating a gradient system according to one of the claims 6 to 9 comprised in the imaging system, when the program is executed in the planning unit (41).

11. Electronically readable data carrier (99) on which a program is stored which is configured such that, on use of the data carrier (99) in a planning unit (41) comprised in an imaging system according to one of claims 4 to 5, the program carries out the method for actuating a gradient system according to one of the claims 6 to 9.

## Revendications

1. Système d'imagerie comprenant un premier appareil (11) à résonnance magnétique, un deuxième appareil (12) à résonnance magnétique et un système à gradient, comprenant une première unité (21) de bobine à gradient, une deuxième unité (22) de bobine à gradient et une unité (35) flexible d'amplificateur de gradient à utiliser conformément au besoin, dans lequel l'unité (35) flexible d'amplificateur de gradient est constituée pour une commande de la première unité (21) de bobine à gradient et de la deuxième unité (22) de bobine à gradient, dans lequel le premier appareil (11) à résonnance magnétique est affecté à la première unité (21) de bobine à gradient et le deuxième appareil (12) à résonnance magnétique est affecté à la deuxième unité (22) de bobine à gradient.

2. Système d'imagerie suivant la revendication 1,
dans lequel le système à gradient a une unité (41) de planification comprenant une entrée (42) de planification,
l'entrée (42) de planification est constituée pour détecter une première information de commande sur une commande de la première unité (21) de bobine à gradient à un premier instant et/ou une deuxième information de commande sur une commande de la deuxième unité (22) de bobine à gradient à un deuxième instant, et l'unité (41) de planification est constituée pour, sur la base de la première information de commande et/ou de la deuxième information de commande, effectuer une affectation de l'unité (35) flexible d'amplificateur de gradient au premier instant et/ou au deuxième instant à la première unité (21) de bobine à gradient et/ou à la deuxième unité (22) de bobine à gradient.

3. Système d'imagerie suivant la revendication 2,
dans lequel l'unité (41) de planification comprend une unité (43) de contrôle, laquelle unité (43) de contrôle est constituée pour détecter au moins une première spécification pour la première unité (21) de bobine à gradient et pour s'assurer que, lors de la commande de la première unité (21) de bobine à gradient par l'unité (35) flexible d'amplificateur de gradient, la première spécification soit respectée.

4. Système d'imagerie suivant l'une des revendications précédentes,
dans lequel le système à gradient comprend une première unité (31) statique d'amplificateur de gradient, reliée à la première unité (21) de bobine à gradient et constituée pour la commande de seulement la première unité (21) de bobine à gradient, et une deuxième unité (32) statique d'amplificateur de gradient reliée à la deuxième unité (22) de bobine à gradient et constituée pour la commande de seulement la deuxième unité (22) de bobine à gradient.

5. Système d'imagerie suivant la revendication 4,
dans lequel une puissance pouvant être produite au maximum par l'unité (35) flexible à amplificateur de gradient est plus grande qu'une puissance pouvant être produite au maximum par la première unité (31) statique à amplificateur de gradient et une puissance pouvant être produite au maximum par l'unité (35) flexible à amplificateur de gradient est plus grande qu'une puissance pouvant être produite au maximum par la deuxième unité (32) statique à amplificateur de gradient.

6. Procédé de commande d'un système à gradient compris dans un système d'imagerie suivant l'une des revendications 4 à 5, selon les stades de procédé suivants :
- mise à disposition d'une première information de commande sur une commande de la première unité (21) de bobine à gradient à un premier instant,
- détermination d'une première puissance nécessaire pour la première unité (21) de bobine à gradient sur la base de la première information de commande,
- analyse comprenant une première comparaison de la première puissance nécessaire à une puissance pouvant être produite au maximum par la première unité (31) statique à amplificateur de gradient,
- affectation de la première unité (35) flexible à amplificateur de gradient à la première unité (21) de bobine à gradient et/ou à la deuxième unité (22) de bobine à gradient et/ou dans une position de repos en fonction de l'analyse.

7. Procédé suivant la revendication 6,
dans lequel l'affectation comprend un décalage dans le temps du premier instant.

8. Procédé suivant l'une des revendications 6 à 7,
dans lequel le procédé comprend, en outre, les stades de procédé suivants :
- mise à disposition d'une deuxième information de commande sur une commande de la deuxième unité (22) de bobine à gradient à un deuxième instant,
- détermination d'une deuxième puissance nécessaire pour la deuxième unité (22) de bobine à gradient sur la base de la deuxième information de commande,
et l'analyse comprend une deuxième comparaison de la deuxième puissance nécessaire à une puissance pouvant être produite au maximum par la deuxième unité (31) statique à amplificateur de gradient et s'effectue en tenant compte du premier instant et du deuxième instant.

9. Procédé suivant la revendication 8,
dans lequel l'affectation comprend un décalage dans le temps du deuxième instant.

10. Produit de programme d'ordinateur, qui comprend un programme et qui peut être chargé directement dans une mémoire d'une unité (41) de planification programmable et comprise dans un système d'imagerie suivant l'une des revendications 4 à 5, comprenant des moyens de programme pour effectuer un procédé de commande d'un système de gradient compris dans le système d'imagerie suivant l'une des revendications 6 à 9 lorsque le programme est exécuté dans l'unité (41) de planification.

11. Support (99) de données déchiffrables électroniquement, sur lequel est mis en mémoire un programme, qui est constitué de manière à ce que le programme effectue lors de l'utilisation du support (99) de données dans une unité (41) de planification comprise dans un système d'imagerie suivant l'une des revendications 4 à 5, le procédé de commande du système d'imagerie suivant l'une des revendications 6 à 9.
